# FASCICULE DE BREVET EUROPEEN

(11) **EP 2 234 598 B1**
(45) Date de publication et mention de la délivrance du brevet: **19.11.2025**
(21) Numéro de dépôt: 08867946.9
(22) Date de dépôt: 18.12.2008
(51) Int. Cl.: A61K 9/08, A61K 47/02, A61K 9/00, A61K 9/20, A61P 3/10, A61P 35/00, A61K 38/00

(54) **COMPOSITIONS PHARMACEUTIQUES RENFERMANT AU MOINS UN PRINCIPE ACTIF PROTEINIQUE PROTEGE DES ENZYMES DIGESTIVES**
PHARMAZEUTISCHE ZUSAMMENSETZUNGEN MIT MINDESTENS EINEM PROTEINÖSEN WIRKSTOFF, DER GEGEN VERDAUUNGSENZYME GESCHÜTZT IST
PHARMACEUTICAL COMPOSITIONS CONTAINING AT LEAST ONE PROTEINACEOUS ACTIVE INGREDIENT PROTECTED AGAINST DIGESTIVE ENZYMES

(30) Priorité: 19.12.2007 FR 0759971
(43) Date de publication de la demande: 06.10.2010
(73) Titulaire: Bennis, Farid, Casablanca 21000 (MA)
(72) Inventeur: BENNIS, Farid, Casablanca 21000 (MA); SERRANO, Jean-Jacques, F-34090 Montpellier (FR)
(74) Mandataire: Cabinet Beau de Loménie
(86) Numéro de dépôt international: PCT/FR2008/052357
(87) Numéro de publication internationale: WO 2009/083686

(56) Documents cités:
- EP-A1- 1 424 077
- WO-A1-02/072075
- WO-A1-2007/032018
- WO-A1-97/33531
- WO-A2-01/36656
- FR-A1- 2 123 524
- US-A- 4 692 433
- US-A1- 2002 132 757
- US-A1- 2003 017 203
- US-A1- 2007 154 559

## Description

La présente invention a pour principal objet des compositions, compositions pour utilisation comme médicaments ou compositions pharmaceutiques, renfermant au moins un principe actif protéinique protégé des enzymes digestives. Au sein desdites compositions, ledit au moins un principe actif est formulé de façon à résister, en l'état, à sa métabolisation en milieu gastrique et en milieu intestinal. Lesdites compositions sont des compositions pour administration par voie orale *(via* le tractus gastro-intestinal) dudit au moins un principe actif protéinique (sensible aux enzymes digestives).

A ce jour, les principes actifs protéiniques, donc sensibles aux enzymes digestives, notamment l'insuline et ses analogues (plus précisément sensibles aux protéases, telle la pepsine dans l'estomac et principalement la trypsine dans l'intestin), sont encore essentiellement administrés par voie parentérale malgré les nombreuses recherches qui ont été menées pour proposer des voies de délivrance alternatives (notamment plus confortables pour les patients).

L'article de Simona Cernea et Itamar Raz, paru dans Timely Top. Med. Cardiovasc. Dis. 2006 Nov. 1 ; Vol 10: E29, faisait, en 2006, un point sur les formes d'administration, alternatives à l'injection, de l'insuline. De nombreux documents brevets, par exemple WO 85/05029, US 5,824,638 et WO 2006/127361, existent également sur le sujet.

Les travaux les plus avancés seraient ceux relatifs à l'administration par voie nasale. Cette voie d'administration serait en effet moins contraignante techniquement que la voie parentérale. La muqueuse nasale, richement vascularisée, présente la capacité d'absorber les protéines et de les transmettre au système sanguin, ce qui en fait un candidat potentiellement intéressant. Cependant, elle souffre d'une certaine difficulté à contrôler la dose délivrée par les inhalateurs en fonction des patients (notamment en cas de rhume...).

Selon l'art antérieur, on a plus généralement décrit des principes actifs protéiniques modifiés chimiquement, des principes actifs protéiniques formulés, selon de nombreuses variantes. Ainsi :
- le brevet US 4,692,433 (D9) décrit l'administration par voie orale d'hormones polypeptidiques. Lesdites hormones sont administrées, avantageusement en solutions aqueuses tamponnées, encapsulées dans un liposome. Elles ne sont pas administrées sous forme libre ;
- les documents WO 97/33531 (D3), WO 02/072075 (D5) et US 2003/0017203 (D4) décrivent des formes gastro-résistantes pour l'administration par voie orale de peptides. Ces formes associent un enrobage gastro-résistant et un agent réducteur de pH. Ledit enrobage protège le principe actif lors de son passage dans l'estomac. Une fois arrivé dans le compartiment intestinal, ledit enrobage est dissous, libérant à la fois le principe actif et l'agent réducteur de pH. De part l'action dudit agent réducteur de pH, le pH de l'intestin est localement abaissé, réduisant de fait l'activité protéolytique des protéases intestinales présentes. La protection au niveau de l'estomac et à l'entrée de l'intestin est donc assurée par deux moyens différents, qui développent successivement leur action. Les peptides en cause n'interviennent ni sous forme libre, ni en présence d'un tampon. On peut incidemment noter ici que le tableau 1, présenté en page 23 de la demande WO 97/33531, montre des résultats de biodisponibilité de solutions tamponnées de calcitonine. Les tests ont été réalisés pour étudier l'influence du pH de la solution administrée localement (directement dans l'intestin de rats) sur l'absorption du principe actif. Ces tests ont été réalisés en vue d'optimiser la nature de l'agent réducteur de pH intervenant dans la forme gastro-résistante proposée. Ces tests ne décrivent, ni ne suggèrent, les compositions (compositions pharmaceutiques ou médicaments) orales de l'invention, décrites ci-après ;
- la demande US 2002/0132757 (D1) concerne l'administration de la calcitonine, sous la forme de particules solides, au travers de membranes épithéliales, au travers des muqueuses buccales ou nasales. Pour ce type spécifique d'administration, qui n'implique pas de tractus gastro-intestinal, le principe actif est traité comme suit. Il est préalablement dissous dans un tampon (simple auxiliaire de fabrication). La solution obtenue, additionnée de tensio-actif(s) et agent(s) améliorant l'absorption est lyophilisée. Les particules sèches obtenues sont finalement conditionnées dans un container sous pression avec un solvant ou véhicule adéquat (éthanol, par exemple). Ce solvant ou véhicule a pour fonction de disperser sous pression lesdites particules sur une surface, la plus grande possible, des muqueuses. Lesdites particules ne sont pas administrées en présence d'un tampon ;

- la demande US 2007/0154559 (D2) décrit un procédé compliqué de formulation de principes actifs, destinés à l'administration par voie orale. Une absorption gastro-intestinale améliorée est recherchée. L'absorption en cause est celle de nanoparticules qui renferment lesdits principes actifs. Selon le procédé décrit, le principe actif est tout d'abord dissous dans un tampon (simple auxiliaire de fabrication) puis complexé avec un contre-ion. Le complexe obtenu est mis en solution, en présence d'un polymère et d'un lipide, dans un solvant organique. Une émulsion est ensuite générée entre la solution organique obtenue et une solution aqueuse contenant un agent émulsifiant. Les nanoparticules sont finalement formées par évaporation dudit solvant organique. Le principe actif n'est ainsi administré, ni sous forme libre, ni en présence d'un tampon ;
- la demande WO 2007/032018 (D8) décrit un procédé compliqué de formulation de principes actifs, destinés à l'administration par voie orale, du même type que celui décrit dans la demande US ci-dessus. Le principe actif est également délivré sous forme de nanoparticules. Lesdites nanoparticules (à base d'un acide gras et d'un polymère) sont sensibles au pH. Elles se rétractent à pH acide. Le principe actif se trouve ainsi mieux protégé lors de son passage dans l'estomac. Ledit principe actif n'est ici encore administré, ni sous forme libre, ni en présence d'un tampon ;
- la demande FR 2 123 524 (D6) décrit un dérivé d'insuline obtenu par acylation. La réaction chimique en cause est mise en œuvre en milieu tampon. La demande WO 01/36656 (D7) décrit un complexe biomolécule/acide hyaluronique. Ces deux documents de l'art antérieur ne décrivent, ni ne suggèrent, des compositions pharmaceutiques associant leur principe actif sous forme libre et un système tampon protecteur.

On a ci-dessus rendu compte de l'enseignement des documents de l'art antérieur évoqués, en référence aux notions de composition orale, de forme libre du principe actif protéinique et de tampon, dans la mesure où lesdites notions constituent le fondement de la présente invention, décrite ci-après.

Le problème technique de l'administration par voie orale d'un principe actif protéinique (donc sensible aux enzymes digestives) est double dans la mesure où le système de protection proposé doit *a priori* être efficace tant au niveau de l'estomac qu'à l'entrée de l'intestin. Il doit *a priori* résister tout d'abord au suc gastrique, puis ensuite au suc pancréatique. En effet, à la sortie de l'estomac, au niveau du pylore, lorsque le chyme acide se déverse dans le duodénum, la sécrétine est libérée de l'intestin et va stimuler le pancréas pour d'une part la sécrétion de bicarbonate (afin de diminuer l'acidité dudit chyme) et d'autre part la sécrétion de la cholécystokinine, pancréoenzyme qui elle, stimule la sécrétion du suc pancréatique riche en enzymes (tripsinogène, chymotrypsinogène, transformées en trypsine et chymotrypsine activées par une entérokinase). Une fois l'acidité neutralisée dans le duodénum par la sécrétine hydrocarbonatée, il y a alors rétrocontrôle et inhibition des sécrétions pancréatiques. Ce mécanisme normal de la digestion est familier à l'homme du métier.

Confrontés audit problème technique de l'administration par voie orale d'un principe actif protéinique, les inventeurs proposent une solution tout à fait originale, non pas basée sur un système de double protection mais sur un système de protection au niveau de l'estomac qui inhibe également la sécrétion pancréatique (qui supprime le problème de la dégradation du principe actif au niveau de l'entrée de l'intestin). Les inventeurs proposent *a posteriori* cette explication des bons résultats obtenus avec les compositions de l'invention. Le système de protection original proposé est un système tampon. De façon tout à fait surprenante, ledit système de protection original, système tampon, autorise l'administration, par voie orale *(via* le tractus gastro-intestinal), du principe actif protéinique, sous forme libre.

La présente invention concerne donc de nouvelles compositions pour utilisation comme médicament ou compositions pharmaceutiques, destinées à (convenant à) l'administration par voie orale d'au moins un principe actif protéinique, tamponnées.

Plus précisément, les compositions de l'invention se présentent sous forme liquide ou solide. Il s'agit de compositions orales et elles renferment au moins un principe actif protéinique. Elles conviennent à l'administration par voie orale dudit principe actif.

De façon caractéristique, lesdites compositions :
- liquides, renferment un système (système tampon) capable de les tamponner à un pH supérieur à 4 et inférieur ou égal à 8 ;
- solides, renferment un système (système tampon) susceptible d'exercer, lors de leur mise en milieu liquide, généralement aqueux, un effet tampon entre un pH supérieur à 4 et un pH inférieur ou égal à 8.

Selon son premier objet, la présente invention concerne donc :
- des compositions, sous forme liquide ou solide, qui renferment :
   au moins un principe actif protéinique sous forme libre, ainsi que, liquides, un système (système tampon) capable de les tamponner à un pH supérieur à 4 et inférieur ou égal à 8 ou, solides, un système (système tampon) susceptible d'exercer, lors de leur mise en milieu liquide, un effet tampon entre un pH supérieur à 4 et un pH inférieur ou égal à 8,
   pour utilisation comme médicaments, pour administration par voie orale (*via* le tractus gastro-intestinal) dudit au moins un principe actif protéinique, ledit au moins un principe actif protéinique étant choisi parmi (i) l'insuline, ses analogues et dérivés, (ii) la somatotropine, (iii) la calcitonine, et (iv) les analogues de la L.H.R.H.;
- des compositions pharmaceutiques, sous forme liquide ou solide, pour administration par voie orale *(via* le tractus gastro-intestinal), renfermant au moins un principe actif protéinique, qui renferment ledit principe actif protéinique, sous forme libre, ainsi que, liquides, un système (système tampon) capable de les tamponner à un pH supérieur à 4 et inférieur ou égal à 8, ou, solides, un système (système tampon) susceptible d'exercer, lors de leur mise en milieu liquide, un effet tampon entre un pH supérieur à 4 et un pH inférieur ou égal à 8, ledit au moins un principe actif protéinique étant choisi parmi (i) l'insuline, ses analogues et dérivés, (ii) la somatotropine, (iii) la calcitonine, et (iv) les analogues de la L.H.R.H..

Lesdites compositions de l'invention sont susceptibles d'être obtenues par (simple) formulation, dudit au moins un principe actif protéinique sous forme libre, avec un système (système tampon) capable de les tamponner, sous forme liquide, à un pH supérieur à 4 et inférieur ou égal à 8 ou, sous forme solide, avec un système (système tampon) susceptible d'exercer, lors de leur mise en milieu liquide de ladite forme solide, un effet tampon entre un pH supérieur à 4 et un pH inférieur ou égal à 8.

De façon caractéristique, les compositions de l'invention, liquides ou solides (monophasiques, en tout état de cause) sont des compositions orales, qui associent en leur sein ledit au moins un principe actif protéinique sous forme libre et un système tampon. Ledit système tampon permet, comme indiqué ci-dessus, l'administration par voie orale du principe actif protéinique, sous forme libre. Il est efficace pour protéger ladite forme libre lors du tractus gastro-intestinal.

Au sein des compositions de l'invention, le principe actif protéinique est donc présent, "en l'état", non protégé *per se,* notamment par une barrière physique... Il est présent tel que ou en simple mélange avec des excipients nécessaires à sa formulation. Par forme libre dudit principe actif, on entend notamment ledit principe actif sans système physique de protection, plus ou moins complexe, type revêtement, enrobage, matrice, paroi de capsule (ledit principe actif n'est ni revêtu, ni enrobé, ni matricé, ni encapsulé (notamment dans des liposomes)...).

Le système tampon, au vu des valeurs de pH énoncées, est capable de tamponner les compositions de l'invention en milieu gastrique et en milieu intestinal. Il est évidemment capable d'exercer son effet tampon pendant la durée de la digestion : pendant au moins 2 h, avantageusement jusqu'à 3 h (dans les conditions acides de l'estomac et basiques de l'intestin). L'homme du métier connaît de tels systèmes tampon. On en précise, à titre nullement limitatif, la nature ci-après.

Les compositions de l'invention associent :
- au moins un principe actif protéinique sous forme libre (voir ci-dessus), généralement un tel principe actif (mais l'intervention conjuguée de plusieurs principes actifs de ce type (voire d'au moins un principe actif de ce type et d'au moins un autre principe actif), en mélange ou séparément, n'est pas exclu du cadre de l'invention) ; et
- un système (système tampon) capable d'exercer un effet tampon dans la zone de pH énoncée ci-dessus.

L'exercice dudit effet tampon dans ladite zone de pH (4 < pH ≤ 8) est évidemment compatible avec la stabilité dudit au moins un principe actif protéinique (en tout état de cause avec la stabilité du(des) principe(s) actif(s) présent(s)).

Les compositions de l'invention sont tamponnées à un pH : 4 < pH ≤ 8. Elles sont avantageusement tamponnées à un pH compris entre 4,5 et 7,5 (4,5 ≤ pH ≤ 7,5), très avantageusement tamponnées à un pH compris entre 5 et 7 (5 ≤ pH ≤ 7), voire à un pH supérieur à 5 et inférieur ou égal à 7 (5 < pH ≤ 7). De façon particulièrement préférée, elles sont tamponnées à un pH de 6,5 ou voisin de 6,5 (6,5 ± 0,2). Cette valeur est tout particulièrement préférée dans le contexte où la composition de l'invention renferme de l'insuline.

Les compositions de l'invention renferment au moins un principe actif protéinique choisi parmi (i) l'insuline, ses analogues et dérivés, (ii) la somatotropine, (iii) la calcitonine, et (iv) les analogues de la L.H.R.H., qu'elles sont capables de protéger des enzymes digestives. On vient de mentionner, à titre de tel principe actif sensible aux enzymes digestives, l'insuline. L'invention a tout particulièrement été développée en référence à ce principe actif (voir les exemples et essais présentés plus avant dans le présent texte). Le mécanisme *a priori* en jeu (que les inventeurs proposent *a posteriori*) *-* protection lors du passage dans l'estomac (à un pH > 4, la pepsine n'est plus (ou quasiment plus) active) et inhibition (plus ou moins conséquente) des sécrétions pancréatiques dans la mesure où ce n'est plus un chyme acide qui se déverse dans le duodénum - convient pour protéger lesdits principes actifs protéiniques des enzymes digestives.

Ainsi, les compositions de l'invention renferment-elles, avantageusement, au moins un principe actif protéinique (sous forme libre) choisi parmi l'insuline, ses analogues et dérivés (renferment-elles généralement avantageusement de l'insuline ou l'un de ses analogues ou dérivés à titre d'unique principe actif de ce type, voire à titre d'unique principe actif). L'homme du métier connaît les analogues de l'insuline tels, par exemple, l'insuline Lipro, l'insuline Aspart, l'insuline Glargine et l'insuline Detemir. Il connaît aussi les dérivés de l'insuline, tels ceux décrits dans la demande FR 2 123 524.

Ainsi, les compositions de l'invention renferment-elles, à titre de principe actif (sous forme libre) :
- l'insuline, l'un de ses analogues ou dérivés,
- la somatotropine (hormone de croissance humaine),
- la calcitonine, ou
- un analogue de la L.H.R.H. ("Luteinising Hormone Releasing Hormone"), tel que la tryptoréline.
Selon la présente description, les compositions renferment, à titre de principe actif (sous forme libre) la somatotropine (hormone de croissance humaine) ou l'un de ses dérivés.

Les systèmes tampons convenant aux fins de l'invention sont des systèmes tampons classiques, avantageusement de forte capacité. L'homme du métier connaît de tels systèmes et est à même d'optimiser une association au sens de l'invention : au moins un principe actif protéinique (sous forme libre)/système tampon (par exemple : insuline/système tampon).

De façon nullement limitative, on peut indiquer que le système responsable de l'effet tampon au sein des compositions de l'invention est avantageusement un tampon choisi parmi les tampons phosphate, acétate, maléate, phtalate, succinate, citrate, imidazole, tétrabutylammonium, 2-amino-2-hydroxyméthyl-1,3-propanediol (ou trihydroxyméthylaminométhane ou Trométamol ou Tham ou Tris), tris-glycine, barbitol, tris-EDTA BSA, sulfate de cuivre et zwittérionique.

De manière plus générale, le système tampon des compositions de l'invention peut être choisi parmi la liste des systèmes tampons donnée dans la Pharmacopée Européenne, édition en cours (monographie 4.1.3).

Ledit système tampon est avantageusement un tampon phosphate ou du Tris.

A titre de tampon phosphate, on préconise un tampon phosphate qui renferme :
de 2 à 3 % en masse de phosphate monosodique dihydrogéné, et
de 97 à 98 % en masse de phosphate disodique monohydrogéné
   qui renferme avantageusement :
   environ 2,8 % en masse de phosphate monosodique dihydrogéné, et
   environ 97,2 % en masse de phosphate disodique monohydrogéné.

Les compositions orales de l'invention (associant de façon originale au moins un principe actif protéinique, sous forme libre, et le système tampon sélectionné : 4 < pH ≤ 8) peuvent exister selon deux variantes.

Selon une première variante, plus classique, elles sont formulées sous forme unitaire. Tous les ingrédients constitutifs, y compris le système tampon, sont formulés ensemble. Dans le cadre de cette première variante, de nombreuses possibilités existent. Les compositions de l'invention peuvent notamment se présenter sous des formes liquides (directement tamponnées à un pH adéquat) telles des solutés, des suspensions et des sirops ou sous des formes solides (qui développent l'effet tampon, lors de leur prise, dans un liquide, généralement de l'eau, ou suite à leur prise, dans l'estomac) telles des comprimés (classiques (à avaler), à sucer, sublinguaux, dispersibles, orodispersibles, effervescents), des gélules, des poudres, des poudres effervescentes, des granulés, des granulés effervescents et des lyophilisats. Ces listes ne sont pas exhaustives. Le galéniste sait formuler, sous l'une ou l'autre des formes unitaires listées ci-dessus, le principe actif en cause avec un système adéquat, responsable de l'effet tampon recherché.

Dans la préparation de formes galéniques effervescentes, il convient d'ajouter les ingrédients aptes à conférer le caractère effervescent escompté. Ce type d'ingrédients (des réactifs (généralement deux réactifs) susceptibles de réagir en dégageant du gaz) est familier à l'homme du métier.

Dans le cadre de cette première variante, les compositions de l'invention se présentent avantageusement sous la forme de préparations galéniques solides, notamment de comprimés dispersibles ou de comprimés effervescents.

Selon une seconde variante, les compositions de l'invention sont des compositions à au moins deux composants séparés, notamment des compositions qui comprennent séparément :
- un composant renfermant le au moins un principe actif protéinique sous forme libre ; et
- un autre composant renfermant au moins le système générateur de l'effet tampon désiré.

Ces deux composants, séparés, sont à administrer conjointement ou quasi conjointement, de sorte, bien évidemment, que l'effet tampon se développe lors du passage du principe actif dans les voies digestives (tout d'abord l'estomac).

Les compositions de l'invention (selon la première ou la seconde des variantes ci-dessus) qui renferment ledit au moins un principe actif protéinique sous forme libre (voire ledit au moins un principe actif protéinique sous forme libre et au moins un autre principe actif) et le système tampon associé, généralement dans un excipient pharmaceutiquement acceptable (avec, si nécessaire, les ingrédients aptes à les rendre effervescentes), sont bien évidemment susceptibles de renfermer d'autres ingrédients, intervenant de façon classique en galénique, du type édulcorant, arôme et/ou auxiliaires de fabrication (lubrifiant...)... Les compositions liquides peuvent ne renfermer que ledit au moins un principe actif protéinique (voire ledit au moins un principe actif protéinique sous forme libre et au moins un autre principe actif) et le système tampon adéquat. Elles renferment généralement, en sus de ces deux composants, des ingrédients de formulation classiquement utilisés en galénique (type ingrédients listés ci-dessus). Les compositions solides renferment généralement, en sus dudit au moins un principe actif protéinique (voire en sus dudit au moins un principe actif protéinique sous forme libre et d'au moins un autre principe actif) et du système tampon, un excipient solide (base, avec éventuellement des ingrédients responsables d'effervescence)) avec divers additifs (type ingrédients listés ci-dessus).

La préparation des compositions de l'invention, unitaires ou non, telles que décrites ci-dessus, est également décrite dans la présente description. Ladite préparation est une préparation de forme galénique tamponnée ou associée à un tampon. De façon caractéristique, elle comprend la (simple) formulation d'au moins un principe actif protéinique sous forme libre, avec un système (système tampon) capable de tamponner ladite composition sous forme liquide (notamment en milieu gastrique et en milieu intestinal) à un pH supérieur à 4 et inférieur ou égal à 8 ou sous forme solide, avec un système (système tampon) susceptible d'exercer, lors de la mise en milieu liquide, notamment aqueux, de ladite forme solide (notamment en milieu gastrique et en milieu intestinal), un effet tampon entre un pH supérieur à 4 et un pH inférieur ou égal à 8. Le terme formulation est à prendre au sens classique du terme (en galénique) pour la préparation de compositions unitaires, au sens plus large (formulation = conditionnement) pour la préparation de compositions à composants séparés.

De façon classique, d'autres ingrédients sont susceptibles d'intervenir dans la préparation des compositions de l'invention.

L'homme du métier a saisi tout l'intérêt de la présente invention, confirmé par les exemples et résultats de tests présentés ci-après. Le "double effet positif" du tampon - gastro-protection et inhibition des sécrétions pancréatiques - est particulièrement efficace. Ce double effet et son efficacité sont réellement surprenants.

Selon un autre de ses aspects, l'invention offre un débouché original à des systèmes tampons et concerne donc aussi l'utilisation d'un système tampon tel que précisé ci-dessus, notamment choisi parmi ceux identifiés ci-dessus, pour la protection d'au moins un principe actif protéinique sous forme libre, lors de son transit *via* le tractus gastro-intestinal, ledit au moins un principe actif protéinique étant choisi parmi (i) l'insuline, ses analogues et dérivés, (ii) la somatotropine, (iii) la calcitonine, et (iv) les analogues de la L.H.R.H.. En d'autres termes, l'invention propose une méthode de protection originale, vis-à-vis des enzymes digestives (lors du transit gastro-intestinal), desdits principes actifs protéiniques. Ladite méthode comprend essentiellement la formulation, sous forme unitaire ou non, desdits principes actifs sous forme libre, avec un système tampon, tel que précisé ci-dessus, notamment choisi parmi ceux identifiés ci-dessus.

La présente description décrit également une méthode de traitement thérapeutique comprenant l'administration par voie orale d'au moins un principe actif protéinique et/ou une méthode d'administration par voie orale d'au moins un tel principe actif. De façon caractéristique, dans le cadre de ladite méthode, ledit au moins un principe actif est administré (formulé dans une composition solide ou liquide, unitaire ou non), sous forme libre, avec un système tampon tel que précisé ci-dessus ; c'est-à-dire tamponné à un pH tel que précisé ci-dessus : 4 < pH ≤ 8 (dans le cas d'une forme liquide) ou susceptible d'être tamponné, lors de sa mise en milieu liquide, à un tel pH (dans le cas d'une forme solide). Les thérapies concernées, connues à ce jour, sont celles des maladies ci-après : le diabète (en référence à l'administration d'insuline), l'inhibition de la croissance (en référence à l'administration de la somatotropine), l'ostéoporose (en référence à l'administration de la calcitonine), le cancer de la prostate (en référence à l'administration des analogues de la L.H.R.H.)...

On se propose maintenant d'illustrer l'invention en précisant la formule de deux comprimés d'insuline tamponnée selon l'invention ; et de démontrer le grand intérêt de ladite invention en présentant ci-après des résultats comparatifs de tests physico-chimiques réalisés *in vitro* et de tests pharmacologiques réalisés *in vivo* avec l'insuline.

### I Formules

On a préparé, de façon connue *per se* (procédé de formulation classique), à partir des ingrédients indiqués, pris dans les quantités indiquées, deux types de comprimés de l'invention :
- des comprimés A dispersibles ; et
- des comprimés B effervescents

| Comprimés A : | | | | |
|---|---|---|---|---|
| | | insuline humaine | | : 3,5 mg (100 U) |
| | | trométamol (TRIS) | | : 100 mg |
| | | calcium phosphate (dicalcique) | | : 250 mg |
| | | cellulose microcristalline | | : 250 mg |
| | | mannitol | | : 250 mg |
| | | stéarate de magnésium | | : 10 mg |
| | | silice colloïdale | | : 1 mg |
| | | crospovidon | | : 50 mg |
| | | sodium benzoate | | : 30 mg |
| | | talc | | : 10 mg |
| | | acide citrique | 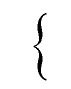 | q.s.p. pH 6,5 |
| | | citrate monosodique | | |
| | | | | poids du comprimé : 1 gramme |

| Comprimés B : | | | | |
|---|---|---|---|---|
| | insuline humaine | | | : 3,5 mg (100 U) |
| | sodium citrate monosodique anhydre | | | : 1142,7 mg |
| | sodium bicarbonate anhydre | | | : 2076 mg |
| | sodium benzoate | | | : 152,60 mg |
| | phosphate monosodique | | | : 120 mg |
| | éthanol 96 % | | 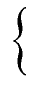 | q.s. pour granulation |
| | eau déminéralisée | | | |
| | | pour un comprimé de masse théorique : 3,5 g | | |
| | | en solution pH : 6,8. | | |

### II Tests in vitro

Des essais ont été réalisés *in vitro* pour confirmer le rôle métabolique de la pepsine en milieu acide, le rôle métabolique de la trypsine en milieu basique et l'inactivation de l'une et l'autre de ces enzymes digestives en milieu tamponné selon l'invention.

Au cours de ces différents essais, l'insuline a été dosée par chromatographie liquide.

### Essai 1'

### Solution d'insuline humaine (100 U)

+ HCl 0,1 N (50 ml) pH 1
+ agitation à 37°C 1 h/2 h/3 h

| Temps | Teneur en insuline |
|---|---|
| Temps 0 | 99,00 U |
| 1 h | 99,87 U |
| 2 h | 100,13 U |
| 3 h | 100,30 U |

En milieu acide, à pH 1, sans pepsine, l'insuline est stable pendant plus de 3 h à 37°C.

### Essai 2'

### Solution d'insuline humaine (100 U)

+ HCl 0,1 N (50 ml) pH 1
+ pepsine (160 mg)
+ agitation à 37°C 1 h

| Temps | Teneur en insuline |
|---|---|
| Temps 0 | 0 U |
| 1 h | 0 U |

En présence de l'enzyme gastrique (la pepsine), à pH 1, l'insuline est immédiatement dégradée.

### Essai 1 (invention)

### Solution d'insuline humaine (100 U)

+ HCl 0,1 N (50 ml) pH 1
+ pepsine (160 mg)
+ tampon phosphate pH 6,8 (50 ml)
+ agitation à 37°C 1 h/2 h/3 h

| Temps | Teneur en insuline |
|---|---|
| Temps 0 | 101,32 U |
| 1 h | 101,73 U |
| 2 h | 99,72 U |
| 3 h | 101,60 U |

En milieu tamponné à pH 6,8, la pepsine n'est plus activée et l'insuline est stable pendant plus de 3 h à 37°C.

### Essai 3'

### Solution d'insuline humaine (100 U)

+ HCl 0,1 N
+ tampon phosphate pH 8,5
+ agitation à 37°C 1 h/2 h/3 h

| Temps | Teneur en insuline |
|---|---|
| Temps 0 | 100 U |
| 1 h | 99,59 U |
| 2 h | 100,24 U |
| 3 h | 100,48 U |

On a vérifié au cours de cet essai l'efficacité du tampon et le fait, qu'en absence d'enzyme, l'insuline est stable en milieu basique.

### Essais 2a, 2b, 2c (invention)

### Solution d'insuline humaine (100 U)

+ tampon phosphate pH 6 (essai 2a) pH 6,5 (essai 2b) pH 6,8 (essai 2c)
+ trypsine 750 U
+ agitation à 37°C 1 h/2 h

| Temps | Teneur en insuline | | |
|---|---|---|---|
| | pH 6 | pH 6,5 | pH 6,8 |
| Temps 0 | 100 U | 100 U | 98 U |
| 1 h | 92 U | 83 U | 56 U |
| 2 h | 86 U | 72 U | 48 U |

En milieu tamponné aux pHs indiqués, l'effet métabolique de la trypsine est en grande partie atténué.

### Essai 4'

### Solution d'insuline humaine (100 U)

+ tampon phosphate pH 8,5
+trypsine 750 U
+ agitation à 37°C 1 h/2 h

| Temps | Teneur en insuline |
|---|---|
| Temps 0 | 89 U |
| 1 h | 14,5 U |
| 2 h | 1,5 U |

En présence de l'enzyme intestinale (la trypsine), à pH 8,5, l'insuline est fortement dégradée.

L'examen de ces résultats (à pHs basiques) montre que plus le pH augmente vers l'alcalinité, plus la trypsine exerce son effet métabolique.

Le point optimum semble être le pH 6,5 (proche de la neutralité) où 2 h après, malgré la présence de trypsine, on retrouve un pourcentage d'insuline important : 72 %.

### III Tests in vivo

L'activité hypoglycémiante de deux types de comprimés effervescents (avec système tampon de l'invention : comprimés B (voir ci-dessus) et sans système tampon de l'invention : comprimés effervescents témoins (du type des comprimés B mais sans tampon)) a été étudiée chez le rat rendu diabétique (hyperglycémique) par administration de streptozotocine.

La streptozotocine, antibiotique chimiquement apparenté aux nitro-urées, possède des propriétés diabétogènes par destruction des îlots de Langherans du pancréas.

Le test est tout à fait familier à l'homme de métier. Son principe est résumé ci-après.

L'administration par voie intrapéritonéale de 70 mg/kg de streptozotocine mise en solution dans le tampon citrate, à des rats mâles, de souche Wistar, d'un poids moyen de 200 g, provoque chez l'animal, au bout de 72 h, une hyperglycémie sévère, associée à une polyphagie, polydipsie et polyurée.

Les animaux sont répartis en trois lots de huit.
Lot 1 : animaux normaux, non hyperglycémiques, recevant par voie orale à l'aide d'une sonde œsophagienne, 30 unités d'insuline contenues dans un comprimé tamponné à pH 6,8 (comprimé effervescent B (voir ci-dessus)), sous un volume de 10 ml/kg.
Lot 2 : animaux diabétiques, recevant par voie orale à l'aide d'une sonde œsophagienne, 30 unités d'insuline contenues dans un comprimé non tamponné (comprimé effervescent du type des comprimés B mais sans tampon), sous un volume de 10 ml/kg.
Lot 3 : animaux diabétiques, recevant par voie orale à l'aide d'une sonde œsophagienne, 30 unités d'insuline contenues dans un comprimé tamponné à pH 6,8 (comprimé effervescent B (voir ci-dessus)), sous un volume de 10 ml/kg.

Les prises de sang sont effectuées toutes les 15 min pendant 3 h à la queue de l'animal et la glycémie est évaluée à l'aide d'un glucomètre Abbot.

Les résultats sont exprimés dans les tableaux ci-après. Ils sont exprimés en gramme de glucose par litre et également en milliéquivalent (tableau 1), et en pourcentage de diminution de la glycémie (tableau 2).

**Tableau 1: Glycémie (g/l et meq/l)**

| Temps en mn | | 0 | 15 | 30 | 45 | 60 | 75 | 90 | 105 | 120 | 135 | 150 | 165 | 180 |
|---|---|---|---|---|---|---|---|---|---|---|---|---|---|---|
| Lot 1 | g/l | 0,99 ± 0,04 | 0,99 ± 0,03 | 0,98 ± 0,05 | 0,90 ± 0,03 | 0,85 ± 0,04 | 0,82 ± 0,05 | 0,82 ± 0,06 | 0,84 ± 0,06 | 0,91 ± 0,05 | 0,95 ± 0,04 | 0,99 ± 0,03 | 1,01 ± 0,02 | 0,99 ± 0,03 |
| | mmeq /l | 5,50 ± 0,2 | 5,50 ± 0,16 | 5,45 ± 0,3 | 5 ± 0,16 | 4,72 ± 0,2 | 4,56 ± 0,3 | 4,56 0,33 | 4,7 ± 0,33 | 5 ± 0,16 | 5,3 ± 0,2 | 5,50 ± 0,16 | 5,6 ± 0,1 | 5,5 ± 0,16 |
| Lot 2 | g/l | 3,94 ± 0,18 | 3,92 ± 0,16 | 3,91 ± 0,16 | 3,88 ± 0,15 | 3,84 ± 0,16 | 3,81 ± 0,14 | 3,82 ± 0,15 | 3,87 ± 0,13 | 3,91 ± 0,14 | 3,94 ± 0,15 | 3,95 ± 0,15 | 3,95 ± 0,16 | 3,92 ± 0,17 |
| | mmeq /l | 22 ± 1 | 21,7 ± 0,9 | 21,7 ± 0,9 | 21,6 ± 0,8 | 21,3 ± 0,9 | 21,2 ± 0,8 | 21,2 ± 0,8 | 21,5 ± 0,7 | 21,7 ± 0,8 | 21,9 ± 0,8 | 21,9 ± 0,8 | 21,9 ± 0,9 | 21,8 ± 0,9 |
| Lot 3 | g/l | 3,8 ± 0,5 | 3,7 ± 0,5 | 3,2 ± 0,4 | 2,5 ± 0,3 | 2,5 ± 0,4 | 1,76 ± 0,3 | 1,64 ± 0,4 | 1,6 ± 0,4 | 1,65 ± 0,5 | 1,8 ± 0,5 | 2,3 ± 0,6 | 2,78 ± 0,8 | 3,4 ± 0,8 |
| | mmeq /l | 21,1 ± 2,8 | 20,6 ± 2,8 | 17,8 ± 2,2 | 14 ± 1,7 | 11,4 ± 2,2 | 9,8 ± 1,7 | 9,1 ± 2,2 | 8,9 ± 2,2 | 9,2 ± 2,8 | 10 ± 2,8 | 12,8 ± 3,3 | 15,4 ± 4,5 | 19 ± 4,5 |

**Tableau 2: % de diminution de la glycémie**

| Temps en mn | 15 | 30 | 45 | 60 | 75 | 90 | 105 | 120 | 135 | 150 | 165 | 180 |
|---|---|---|---|---|---|---|---|---|---|---|---|---|
| Lot 1 | 0 | 1 | 9 | 14 | 17 | 17 | 15 | 8 | 4 | 0 | 0 | 0 |
| Lot 2 | 0,50 | 0,75 | 1,5 | 2,5 | 3,3 | 3 | 1,8 | 0,75 | 0,50 | 0 | 0 | 0 |
| Lot 3 | 1 | 16 | 33 | 46 | 54 | 56 | 57 | 57 | 52 | 39 | 27 | 11 |

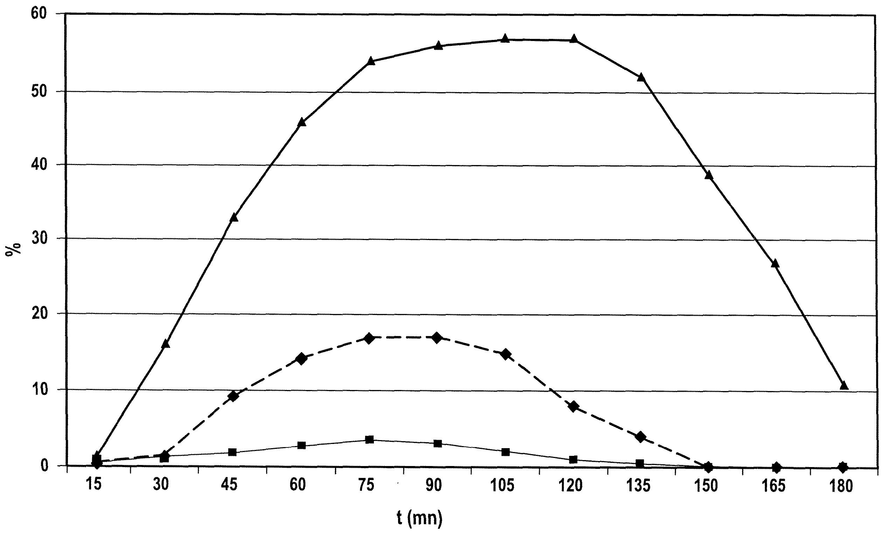

Les résultats du tableau 2 ont été portés sur l'unique figure annexée (pourcentage de diminution de la glycémie en fonction du temps (exprimé en minute)):
- la courbe -■- montre les résultats du lot 1,
- la courbe --◆-- montre les résultats du lot 2,
- la courbe -▲- montre les résultats du lot 3.

L'examen des résultats montre :
- chez l'animal normal (non hyperglycémique) (lot 1), l'administration d'insuline tamponnée entraîne dès 45 min une légère diminution de la glycémie dont le maximum se situe à la 75^{ème} minute, puis la glycémie retrouve des valeurs normales dès la 150^{ème} minute. Ces animaux, à pancréas intact, compensent par sécrétion de glucagon qui est hyperglycémiant ;
- chez l'animal diabétique (lot 2), l'administration d'insuline non tamponnée entraîne une très légère diminution de la glycémie non significative. Durant toute l'expérimentation, les animaux conservent une glycémie très élevée ;
- chez l'animal diabétique (lot 3), l'administration d'insuline tamponnée entraîne dès 45 min une diminution hautement significative de la glycémie dont le maximum se situe à la 105^{ème} minute, puis on observe une remontée progressive de la glycémie. Tous les animaux sont fortement corrigés, bien qu'ils ne retrouvent pas une glycémie normale, due à la sévérité du diabète.

Ces résultats montrent que le système tampon utilisé a permis de conserver l'activité hypoglycémiante de l'insuline administrée par voie orale (ce qui est tout à fait compatible avec les résultats obtenus *in vitro*) et confirment une bonne biodisponibilité de ladite insuline.

Les résultats, tant *in vitro* que *in vivo,* démontrent l'efficacité du système tampon pour préserver l'activité de l'insuline :
- *in vitro,* en milieu acide, non tamponné, l'insuline est métabolisée par la pepsine (essai 2'). En milieu tamponné à pH 6,8, la pepsine n'est plus active et on retrouve de ce fait 100 % d'insuline à l'issue de 3 h (essai 1),
   en milieu basique, l'insuline est métabolisée par la trypsine (essai 4'). En milieux tamponnés à pH 6, 6,5 et 6,8, l'activité de ladite trypsine est inhibée, plus ou moins diminuée ;
- *in vivo,* la préparation non tamponnée ne présente pratiquement pas d'activité. La préparation tamponnée présente par contre une importante activité hypoglycémiante.

Dans tous les essais, tant *in vitro* que *in vivo,* l'insuline humaine a été employée. Il est évident que les résultats obtenus sont transposables à tous les analogues de l'insuline.

A la considération de ces résultats, on ne manque pas de saisir tout l'intérêt de la présente invention. Cet intérêt a été confirmé par des essais préliminaires chez l'homme.

## Revendications

1. Composition, sous forme liquide ou solide, renfermant :
au moins un principe actif protéinique sous forme libre, ainsi que, liquide, un système capable de la tamponner à un pH supérieur à 4 et inférieur ou égal à 8 ou, solide, un système susceptible d'exercer, lors de sa mise en milieu liquide, un effet tampon entre un pH supérieur à 4 et un pH inférieur ou égal à 8,
pour son utilisation comme médicament, dans le traitement d'une maladie par voie orale *via* le tractus gastro-intestinal,
ledit au moins un principe actif protéinique étant choisi parmi (i) l'insuline, ses analogues et dérivés, (ii) la somatotropine, (iii) la calcitonine, et (iv) les analogues de la L.H.R.H..

2. Composition pour utilisation selon la revendication 1, susceptible d'être obtenue par formulation, dudit au moins un principe actif protéinique sous forme libre, avec un système capable de la tamponner, sous forme liquide, à un pH supérieur à 4 et inférieur ou égal à 8 ou, sous forme solide, avec un système susceptible d'exercer, lors de la mise en milieu liquide de ladite forme solide, un effet tampon entre un pH supérieur à 4 et un pH inférieur ou égal à 8.

3. Composition pour utilisation selon l'une quelconque des revendications 1 ou 2, **caractérisée en ce que** le pH tampon est compris entre 4,5 et 7,5, très avantageusement entre 5 et 7.

4. Composition pour utilisation selon l'une quelconque des revendications 1 à 3, **caractérisée en ce que** ledit au moins un principe actif protéinique est choisi parmi l'insuline, ses analogues et dérivés.

5. Composition pour utilisation selon l'une quelconque des revendications 1 à 4, **caractérisée en ce que** ledit système, responsable de l'effet tampon, est un tampon choisi parmi les tampons phosphate, acétate, maléate, phtalate, succinate, citrate, imidazole, tétrabutylammonium, trihydroxyméthylaminométhane, tris-glycine, barbitol, tris-EDTA BSA, sulfate de cuivre et zwittérionique.

6. Composition pour utilisation selon l'une quelconque des revendications 1 à 5, **caractérisée en ce qu'**elle est formulée sous forme unitaire.

7. Composition pour utilisation selon l'une quelconque des revendications 1 à 6, **caractérisée en ce qu'**elle se présente, liquide, sous la forme d'un soluté, d'une suspension ou d'un sirop ou, solide, sous la forme de comprimés, notamment dispersibles, orodispersibles, effervescents, de gélules, d'une poudre, d'une poudre effervescente, de granulés, de granulés effervescents ou d'un lyophilisat.

8. Composition pour utilisation selon l'une quelconque des revendications 1 à 7, **caractérisée en ce qu'**elle se présente sous la forme de préparations galéniques solides, notamment de comprimés dispersibles ou de comprimés effervescents.

9. Composition pour utilisation selon l'une quelconque des revendications 1 à 5, **caractérisée en ce que** ledit système responsable de l'effet tampon est formulé séparément dudit au moins un principe actif protéinique.

10. Utilisation d'un système tampon pour la fabrication d'une composition pharmaceutique destinée au traitement du diabète par voie orale *via* le tractus gastro-intestinal ; ladite composition pharmaceutique, liquide ou solide, renfermant au moins un principe actif protéinique choisi parmi l'insuline, ses analogues et dérivés, sous forme libre ainsi que ledit système tampon ; ledit système tampon convenant pour tamponner ladite composition pharmaceutique, liquide, à un pH compris entre 5 et 7 ou étant susceptible d'exercer, lors de la mise en milieu liquide de ladite composition pharmaceutique solide, un effet tampon à un pH compris entre 5 et 7 ; ledit système tampon protégeant ainsi ledit au moins un principe actif protéinique lors du tractus gastro-intestinal.

11. Utilisation d'un système tampon pour la fabrication d'une composition pharmaceutique destinée au traitement d'une maladie par voie orale *via* le tractus gastro-intestinal; ladite composition pharmaceutique, liquide ou solide, renfermant au moins un principe actif protéinique sous forme libre ainsi que ledit système tampon ; ledit système tampon convenant pour tamponner ladite composition pharmaceutique, liquide, à un pH supérieur à 4 et inférieur ou égal à 8 ou étant susceptible d'exercer, lors de la mise en milieu liquide de ladite composition pharmaceutique solide, un effet tampon entre un pH supérieur à 4 et inférieur ou égal à 8 ; ledit système tampon protégeant ainsi ledit au moins un principe actif protéinique lors du tractus gastro-intestinal,
ledit au moins un principe actif protéinique étant choisi parmi (i) l'insuline, ses analogues et dérivés, (ii) la somatotropine, (iii) la calcitonine, et (iv) les analogues de la L.H.R.H..

## Patentansprüche

1. Zusammensetzung, in flüssiger oder fester Form, die Folgendes einschließt:
mindestens einen Proteinwirkstoff in freier Form sowie, flüssig, ein System, das in der Lage ist, ihn auf einen pH von über 4 und unter oder gleich wie 8 zu puffern, oder, fest, ein System, das in der Lage ist, bei seinem Einbringen in ein flüssiges Medium eine Pufferwirkung zwischen einem pH von über 4 und einem pH von unter oder gleich wie 8 auszuüben,
zur Verwendung als Medikament, zur Behandlung einer Krankheit oral über den Magen-Darm-Trakt,
wobei der mindestens eine Proteinwirkstoff ausgewählt ist aus (i) Insulin, seinen Analoga und Derivaten, (ii) Somatotropin, (iii) Calcitonin und (iv) LHRH-Analoga.

2. Zusammensetzung zur Verwendung nach Anspruch 1, die durch Formulierung des mindestens einen Proteinwirkstoffs in freier Form mit einem System erlangt werden kann, das in der Lage ist, sie in flüssiger Form bei einem pH von über 4 und unter oder gleich wie 8 zu puffern, oder in fester Form mit einem System, das bei dem Einbringen der festen Form in ein flüssiges Medium eine Pufferwirkung zwischen einem pH von über 4 und einem pH von unter oder gleich wie 8 ausüben kann.

3. Zusammensetzung zur Verwendung nach einem der Ansprüche 1 oder 2, **dadurch gekennzeichnet, dass** der pH-Puffer zwischen 4,5 und 7,5, sehr vorteilhafterweise zwischen 5 und 7, liegt.

4. Zusammensetzung zur Verwendung nach einem der Ansprüche 1 bis 3, **dadurch gekennzeichnet, dass** der mindestens eine Proteinwirkstoff ausgewählt ist aus Insulin, seinen Analoga und Derivaten.

5. Zusammensetzung zur Verwendung nach einem der Ansprüche 1 bis 4, **dadurch gekennzeichnet, dass** das System, das für die Pufferwirkung verantwortlich ist, ein Puffer ist, der ausgewählt ist aus Phosphat-, Acetat-, Maleat-, Phthalat-, Succinat-, Citrat-, Imidazol-, Tetrabutylammonium-, Trihydroxymethylaminomethan-, Tris-Glycin-, Barbital-, Tris-EDTA-BSA-, Kupfersulfat- und zwitterionischen Puffern.

6. Zusammensetzung zur Verwendung nach einem der Ansprüche 1 bis 5, **dadurch gekennzeichnet, dass** sie in Einheitsform formuliert ist.

7. Zusammensetzung zur Verwendung nach einem der Ansprüche 1 bis 6, **dadurch gekennzeichnet, dass** sie flüssig in Form einer Lösung, einer Suspension oder eines Sirups oder fest in Form von Tabletten, insbesondere dispergierbaren, orodispergierbaren, Brausetabletten, Kapseln, einem Pulver, einem Brausepulver, einem Granulat, einem Brausegranulat oder einem Lyophilisat vorliegt.

8. Zusammensetzung zur Verwendung nach einem der Ansprüche 1 bis 7, **dadurch gekennzeichnet, dass** sie in Form fester galenischer Präparate, insbesondere in Form von dispergierbaren Tabletten oder Brausetabletten, vorliegt.

9. Zusammensetzung zur Verwendung nach einem der Ansprüche 1 bis 5, **dadurch gekennzeichnet, dass** das für die Pufferwirkung verantwortliche System separat von dem mindestens einen Proteinwirkstoff formuliert ist.

10. Verwendung eines Puffersystems zur Herstellung einer pharmazeutischen Zusammensetzung zur Behandlung von Diabetes auf oralem Weg über den Gastrointestinaltrakt, wobei die pharmazeutische Zusammensetzung flüssig oder fest ist und mindestens einen Proteinwirkstoff, ausgewählt aus Insulin, seinen Analoga und Derivaten, in freier Form sowie das Puffersystem einschließt; wobei das Puffersystem geeignet ist, die flüssige pharmazeutische Zusammensetzung bei einem pH zwischen 5 und 7 zu puffern, oder geeignet ist, beim Einbringen der festen pharmazeutischen Zusammensetzung in ein flüssiges Medium eine Pufferwirkung bei einem pH zwischen 5 und 7 auszuüben; wobei das Puffersystem dadurch den mindestens einen Proteinwirkstoff während des Magen-Darm-Trakts schützt.

11. Verwendung eines Puffersystems zur Herstellung einer pharmazeutischen Zusammensetzung zur Behandlung einer Krankheit auf oralem Weg über den Magen-Darm-Trakt; wobei die flüssige oder feste pharmazeutische Zusammensetzung mindestens einen Proteinwirkstoff in freier Form sowie das Puffersystem einschließt; wobei das Puffersystem geeignet ist, die flüssige pharmazeutische Zusammensetzung auf einen pH über 4 und unter oder gleich wie 8 zu puffern, oder geeignet ist, beim Einbringen der festen pharmazeutischen Zusammensetzung in ein flüssiges Medium eine Pufferwirkung zwischen einem pH über 4 und unter oder gleich wie 8 auszuüben; wobei das Puffersystem dadurch den mindestens einen Proteinwirkstoff während des Magen-Darm-Trakts schützt,
wobei der mindestens eine Proteinwirkstoff ausgewählt ist aus (i) Insulin, seinen Analoga und Derivaten, (ii) Somatotropin, (iii) Calcitonin und (iv) LHRH-Analoga.

## Claims

1. Composition, in liquid or solid form, containing:
at least one protein active ingredient in free form, as well as, for a liquid, a system capable of buffering it to a pH greater than 4 and less than or equal to 8 or, for a solid, a system that exerts, when it is placed in a liquid medium, a buffering effect between a pH greater than 4 and a pH less than or equal to 8,
for use as a medicament in the oral treatment of a disease *via* gastrointestinal tract,
said at least one protein active ingredient is chosen from (i) insulin, analogues thereof and derivatives thereof, (ii) somatotropin, (iii) calcitonin, and (iv) L.H.R.H. analogues.

2. Composition for use according to claim 1, obtainable by the formulation of said at least one protein active ingredient in free form, with a system capable of buffering it, in liquid form, to a pH greater than 4 and less than or equal to 8 or, in solid form, with a system that exerts, when said solid form is placed in a liquid medium, a buffer effect between a pH greater than 4 and a pH less than or equal to 8.

3. Composition for use according to any one of claims 1 or 2, **characterized in that** the buffer pH is between 4.5 and 7.5, very advantageously between 5 and 7.

4. Composition for use according to any one of claims 1 to 3, **characterized in that** said at least one protein active ingredient is selected among insulin, analogues thereof and derivatives thereof.

5. Composition for use according to any one of claims 1 to 4, **characterized in that** said system, responsible for the buffer effect, is a buffer chosen among phosphate, acetate, maleate, phthalate, succinate, citrate, imidazole, tetrabutylammonium, trihydroxymethylaminomethane, tris-glycine, barbitol, tris-EDTA BSA, copper sulfate and zwitterionic buffers.

6. Composition for use according to any one of claims 1 to 5, **characterized in that** it is formulated in unit form.

7. Composition for use according to any one of claims 1 to 6, **characterized in that** it is, as a liquid, in the form of a solution, suspension or syrup or, as a solid, in the form of tablets, notably dispersible, orodispersible or effervescent tablets, capsules, powder, effervescent powder, granules, effervescent granules or a lyophilisate.

8. Composition for use according to any one of claims 1 to 7, **characterized in that** it is in the form of solid pharmaceutical preparations, notably dispersible tablets or effervescent tablets.

9. Composition for use according to any one of claims 1 to 5, **characterized in that** said system responsible for the buffer effect is formulated separately from said at least one protein active ingredient.

10. Use of a buffer system for the manufacture of a pharmaceutical composition intended for the oral treatment of diabetes *via* the gastrointestinal tract; said pharmaceutical composition, which may be liquid or solid, containing at least one active protein ingredient selected from insulin, analogues thereof and derivatives thereof, in free form, as well as said buffer system; said buffer system being capable of buffering said liquid pharmaceutical composition to a pH between 5 and 7 or being capable of exerting, when said solid pharmaceutical composition is placed in a liquid medium, a buffering effect at a pH between 5 and 7; said buffer system thus protecting said at least one protein active ingredient in the gastrointestinal tract.

11. Use of a buffer system for the manufacture of a pharmaceutical composition intended for the treatment of a disease by oral administration *via* the gastrointestinal tract; said pharmaceutical composition, liquid or solid, containing at least one active protein ingredient in free form as well as said buffer system; said buffer system being capable of for buffering said liquid pharmaceutical composition at a pH greater than 4 and less than or equal to 8 or being capable of exerting, when said solid pharmaceutical composition is placed in a liquid medium, a buffering effect between a pH greater than 4 and less than or equal to 8; said buffer system thus protecting said at least one protein active ingredient in the gastrointestinal tract, said at least one protein active ingredient being selected from (i) insulin, analogues thereof and derivatives thereof, (ii) somatotropin, (iii) calcitonin, and (iv) L.H.R.H. analogues.
